# EUROPEAN PATENT APPLICATION

(11) **EP 4 253 961 A1**
(43) Date of publication of application: **04.10.2023**
(21) Application number: 23165951.7
(22) Date of filing: 31.03.2023
(51) Int. Cl.: G01N 33/80, B01L 3/00, G01N 15/14, G01N 21/82, G01N 33/49, G01N 15/05, G01N 35/00, G01N 15/00

(54) **BLOOD TEST APPARATUS, BLOOD TEST METHOD, AND BLOOD TEST PROGRAM**

(30) Priority: 31.03.2022 JP 2022058888
(71) Applicant: Nihon Kohden Corporation, Shinjuku-ku Tokyo (JP); Shibaura Institute of Technology, Tokyo 135-8548 (JP)
(72) Inventor: Higuchi, Makoto, Tokorozawa-shi, Saitama (JP); Watanabe, Nobuo, Koto-ku, Tokyo (JP)
(74) Representative: Grünecker Patent- und Rechtsanwälte PartG mbB

(57) **Abstract**

A blood test apparatus (100) includes a blood acquisition unit (110), a reagent addition unit (120), an aggregation measurement unit (190) and a blood type determination unit (190). The blood acquisition unit (110) acquires a blood sample. The reagent addition unit (120) adds a reagent to the blood sample. The aggregation measurement unit (190) measures a degree of erythrocyte aggregation of the blood sample added with the reagent. The blood type determination unit (190) determines a blood type of the blood sample based on a change in the degree of erythrocyte aggregation of the blood sample added with the reagent. The reagent contains an antibody that generates antigen-antibody reaction with an antigen contained in the blood sample, or an antigen that generates antigen-antibody reaction with an antibody contained in the blood sample. The blood test apparatus (100) can determine the blood type with high accuracy.

## Description

### TECHNICAL FIELD

The presently disclosed subject matter relates to a blood test apparatus, a blood test method, and a blood test program.

### BACKGROUND

In order to prevent in advance side effects due to blood type incompatibility during blood transfusion into a patient in surgery or the like, after confirming a blood type of the patient by ABO blood typing and Rh blood typing, a cross-matching test is performed in advance to confirm a reaction between a blood product for transfusion and blood of the patient. In the ABO blood typing, the ABO blood typing is determined based on results of forward typing and reverse typing. In the forward typing, a blood type determination reagent containing anti-A antibody (hereinafter simply referred to as "reagent") is added to a blood sample collected from the patient (subject), and presence or absence of irreversible agglutination due to an antigen-antibody reaction between A antigen of erythrocytes and the anti-A antibody is confirmed. A reagent containing anti-B antibody is also added to the blood sample, and presence or absence of irreversible agglutination due to an antigen-antibody reaction between B antigen of erythrocytes and anti-B antibody is confirmed.

Then, the blood type is determined based on a combination of the presence or absence of agglutination due to the antigen-antibody reaction between the antigens in the erythrocyte and the anti-A and anti-B antibodies. In the reverse typing, type A blood cells and type B blood cells were separately added to blood plasma of the subject, and presence or absence of irreversible agglutination due to a antigen-antibody reaction was confirmed for the type A and B blood cells in the same manner as in the forward typing, and the blood type is determined based on a combination of the presence or absence of agglutination. As for the Rh blood type, erythrocytes are used in the same manner as in the forward typing to confirm presence or absence of irreversible agglutination due to an antigen-antibody reaction with anti-D antibody, so as to determine Rh-positive or Rh-negative.

Known blood type testing methods in the related art include a test tube method in which the test is performed in a test tube, and a slide method in which the test is performed on a slide glass. However, in these methods, it is necessary to wash the blood cells in order to distinguish agglutination from aggregation called rouleaux, which are formed by reversible aggregation of erythrocytes via blood plasma proteins, and it also takes time and effort to dilute the blood sample. Since a person in charge of the test visually confirms the presence or absence of agglutination, it is necessary to use a sufficient amount of reagent for the agglutination reaction to generate clumps of a size that can be visually distinguished by human eyes, and dilution is also necessary for easy visual determination. Errors may occur due to visual distinguish, and individual differences may also occur in determination due to experience of people in charge. In recent years, there is a column agglutination method in which a blood sample mixed with a reagent is passed through a column filled with beads, and blood type determination is performed by measuring a degree of passage of erythrocytes through the column. However, the column agglutination method requires a dedicated cartridge and a centrifugal mechanism, which poses a problem of increasing a size of an apparatus.

In contrast to the methods in the related art of visually confirming the presence or absence of erythrocyte agglutination, such as the above-described test tube method, there is also known a technique of mechanically detecting the presence or absence of erythrocyte agglutination. For example, JP2016-151417A and JP2015-522825A disclose techniques of mechanically detecting the presence or absence of erythrocyte agglutination and measuring strength of the agglutination by the antigen-antibody reaction. EP1064557A2 and WO99/051982A2 disclose determination of the blood type by evaluating images of deposits resulting from the antigen-antibody reaction and detecting the deposits.

### SUMMARY

However, in the techniques described in JP2016-151417A, JP2015-522825A, EP1064557A2 and WO99/051982A2 when the washing and dilution steps are omitted in order to shorten the blood type test process, or when an amount of the reagent is reduced in order to reduce a cost, it is difficult to distinguish whether the detected agglutinations and deposits are the agglutinations and deposits formed by the antigen-antibody reaction or the rouleaux or deposits thereof formed by reversible aggregation of erythrocytes via blood plasma proteins. Therefore, there is a problem that the blood type cannot be determined with high accuracy.

The presently disclosed subject matter is made in view of the above problem. Accordingly, a main object of the presently disclosed subject matter is to provide a blood test apparatus, a blood test method, and a blood test program capable of omitting the washing and dilution steps in blood type test, reducing the amount of reagents for blood type determination, and determining the blood type with high accuracy.

The above problem of the presently disclosed subject matter is solved by the following configurations.

A blood test apparatus of the presently disclosed subject matter includes a blood acquisition unit, a reagent addition unit, an aggregation measurement unit and a blood type determination unit. The blood acquisition unit acquires a blood sample. The reagent addition unit adds a reagent to the blood sample. The aggregation measurement unit measures a degree of erythrocyte aggregation of the blood sample added with the reagent. The blood type determination unit determines a blood type of the blood sample based on a change in the degree of erythrocyte aggregation of the blood sample added with the reagent.

A blood test method of the presently disclosed subject matter includes a step (a) of controlling to add a reagent to a blood sample, a step (b) of controlling to supply the blood sample added with the reagent to a flow path, a step (c) of measuring a degree of erythrocyte aggregation of the blood sample in the flow path, and a step (d) of determining a blood type of the blood sample based on a change in the degree of erythrocyte aggregation of the blood sample. The reagent contains an antibody that generates antigen-antibody reaction with an antigen contained in the blood sample, or an antigen that generates antigen-antibody reaction with an antibody contained in the blood sample.

According to the presently disclosed subject matter, a degree of reversible aggregation of erythrocytes after adding a reagent containing a blood type antibody or a blood type antigen to a blood sample is measured, and based on change in the degree of aggregation after the addition of the reagent, a blood type of the blood sample is determined. Therefore, it is possible to distinguish the agglutinations due to the antigen-antibody reaction from the rouleaux, so that the blood type can be determined with high accuracy.

### BRIEF DESCRIPTION OF DRAWINGS

FIG. 1 is a block diagram illustrating a schematic configuration of a hemocytometer according to a first embodiment;
FIG. 2 is a schematic diagram illustrating the schematic configuration of the hemocytometer according to the first embodiment;
FIG. 3 is a block diagram illustrating a schematic configuration of a control unit illustrated in FIGs. 1 and 2;
FIG. 4 is a flow chart for explaining an outline of a processing procedure of a blood test method by the hemocytometer illustrated in FIGs. 1 and 2;
FIG. 5 is a subroutine flow chart for explaining measurement processing (S101) of intensity of transmitted light in the flow chart in FIG. 4;
FIG. 6 is a subroutine flow chart for explaining measurement processing (S102/S103) of intensity of transmitted light in the flow chart in FIG. 4;
FIG. 7 is a schematic diagram (syllectogram) illustrating a time course of the intensity of the transmitted light before and after flowing of a blood sample is stopped;
FIGs. 8A to 8H are graphs illustrating measurement results of the intensity of the transmitted light for blood samples of different blood types after flowing of a fluid is stopped;
FIG. 9 is a graph illustrating calculation results of a rate of change in aggregation parameter (AMP) before and after addition of a reagent (with a mixing ratio of 5%);
FIG. 10 is a diagram illustrating a relation between antigen-antibody reaction and blood type in a forward typing.
FIG. 11 is a schematic diagram illustrating a schematic configuration of a hemocytometer according to a second embodiment;
FIG. 12 is a subroutine flow chart for explaining measurement processing (S101) of the intensity of the transmitted light according to the second embodiment; and
FIG. 13 is a subroutine flow chart for explaining measurement processing (S102/S103) of the intensity of the transmitted light according to the second embodiment.

### DESCRIPTION OF EMBODIMENTS

Hereinafter, blood test apparatuses, blood test methods, and blood test programs according to embodiments of the presently disclosed subject matter will be described in detail with reference to the drawings. In the drawings, the same reference numerals are used for the same members. Dimensional ratios in the drawings are exaggerated for convenience of description, and may be different from actual ratios.

### (First Embodiment)

### <Configuration of Hemocytometer>

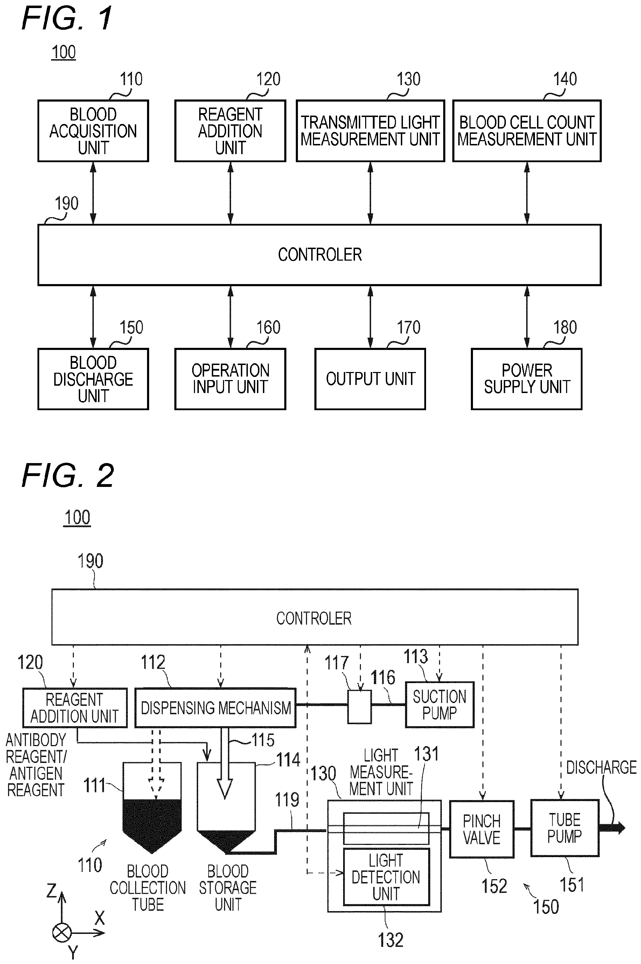

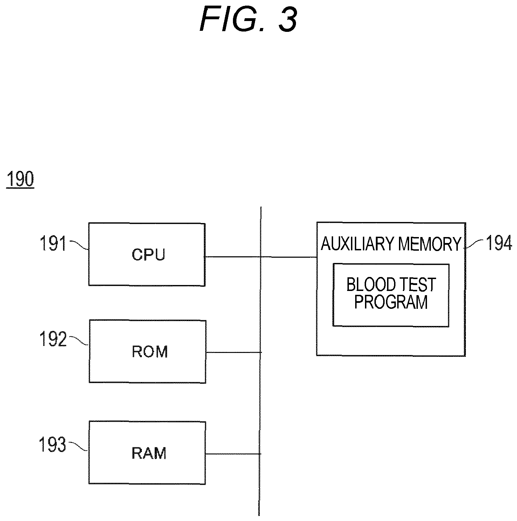

FIGs. 1 and 2 are a block diagram and a schematic diagram illustrating a schematic configuration of a hemocytometer according to a first embodiment, and FIG. 3 is a block diagram illustrating a schematic configuration of a control unit shown in FIGs. 1 and 2.

As shown in FIGs. 1 and 2, a hemocytometer (blood test apparatus) 100 may include a blood acquisition unit 110, a reagent addition unit 120, a light measurement unit 130, a blood cell count measurement unit 140, a blood discharge unit 150, an operation input unit 160, an output unit 170, a power supply unit 180, and a control unit 190. The blood acquisition unit 110, reagent addition unit 120, light measurement unit 130, blood cell count measurement unit 140, blood discharge unit 150, operation input unit 160, output unit 170, and power supply unit 180 are all connected to the control unit 190 and controlled by the control unit 190.

As shown in FIG. 3, the control unit 190 may include a central processing unit (CPU) 191, a read only memory (ROM) 192, a random access memory (RAM) 193, and an auxiliary memory 194. The auxiliary memory 194 may include a solid state drive (SSD) or a hard disk drive (HDD) 194, for example. The CPU 191, ROM 192, RAM 193, and auxiliary memory 194 constitute a computer.

The CPU 191 implements various functions by loading a blood test program stored in advance in the ROM 192 or the auxiliary memory 194 into the RAM 193 and executing the program.

The ROM 192 is a non-volatile memory. An operating system (OS), programs such as the blood test program, and various parameters necessary for arithmetic processing of the CPU 191 are stored in the ROM 192.

The RAM 193 is a volatile memory that temporarily stores a result of the arithmetic processing by the CPU 191 and various data. The auxiliary memory 194 stores the programs such as the blood test program, a measurement result by the light measurement unit 130, the result of the arithmetic processing by CPU 191, and the like.

The blood acquisition unit 110 acquires a blood sample from a blood collection tube 111 set by a medical worker or the like (hereinafter also referred to as a "user") at an inlet (not shown) of the hemocytometer 100, and supplies the acquired blood sample to the light measurement unit 130. The blood acquisition unit 110 may include the blood collection tube 111, a dispensing mechanism 112, a suction pump 113, a blood storage unit 114, a nozzle 115, a pipe 116, an electromagnetic valve 117, and the like.

The blood sample is previously collected from a patient and stored in the blood collection tube 111. In the blood collection tube 111, an anticoagulant such as ethylenediaminetetraacetic acid (EDTA) can be added to the blood sample.

The reagent addition unit 120 adds a reagent to the blood sample stored in the blood storage unit 114 in accordance with a command from the control unit 190. The reagent may be a blood type antibody reagent containing an antibody that generates antigen-antibody reaction with an antigen in erythrocytes contained in the blood sample. Alternatively, the reagent may be a blood type antigen reagent containing an antigen that generates antigen-antibody reaction with an antibody in blood plasma contained in the blood sample. When the reagent is a blood type antibody reagent, a blood type test corresponding to forward typing can be performed, and when the reagent is a blood type antigen reagent, a blood type test corresponding to reverse typing can be performed. Hereinafter, in the present embodiment, a case where the reagent is a blood type antibody reagent will be described as an example.

The reagent addition unit 120 may include a supply mechanism (not shown) that supplies a predetermined amount of the reagent to the blood collection tube 111.

The dispensing mechanism 112 may include the nozzle 115 and a moving mechanism (not shown) that moves the nozzle 115. The dispensing mechanism 112 is connected to the suction pump 113 via the pipe 116. The suction pump 113 is configured to operate a motor according to a command from the control unit 190 to suck the blood sample (for example, approximately 80 [µL]) into the nozzle 115. It should be noted that the blood storage unit 114 may be provided with a supply port for supplying a diluent for diluting the blood sample.

The moving mechanism is a mechanism configured to move the nozzle 115 in X, Y, and Z directions of a rectangular coordinate system, and moves the nozzle 115 to a predetermined position above the blood storage unit 114 by moving the nozzle 115 from a position above the blood collection tube 111 in a vertical up-down direction (the Z direction) and in a horizontal direction (the X and Y directions). Each predetermined position is a position suitable for the nozzle 115 to inject the blood sample into the blood storage unit 114.

The control unit 190 operates the suction pump 113 to suck a fluid in the pipe 116, and opens the electromagnetic valve 117 disposed in the pipe 116 to suck the blood sample in the blood collection tube 111 through the nozzle 115, and then closes the electromagnetic valve 117 to hold the blood sample in the nozzle 115. Then, the control unit 190 moves the nozzle 115 to a predetermined position above the blood storage unit 114, and opens the electromagnetic valve 117 to inject a predetermined amount of the blood sample in the nozzle 115 from a predetermined position into the blood storage unit 114.

The light measurement unit 130 may include a flow path 131, a light detection unit 132, a heater (not shown), and the like, and measures erythrocyte aggregation of the blood sample passing through the flow path 131. The flow path 131 can be, for example, a thin transparent tube (for example, a glass tube with high thermal conductivity or resin) having a inner diameter of approximately 1 [mm]. A cross-sectional shape of the tube can be circular, rectangular, polygonal, or the like. An upstream end portion of the flow path 131 is connected to one end of the pipe 119, and the other end of the pipe 119 is connected to an outlet of the blood storage unit 114. A downstream end portion of the flow path 131 is connected to one end of a pinch valve 152 of the blood discharge unit 150 via a pipe.

During measurement of transmitted light or reflected light from the flow path 131, the heater of the light measurement unit 130 adjusts a temperature of the blood sample flowing through the flow path 131 to a constant temperature, for example, to 37°C (a temperature near human body temperature) suitable for antibody reaction. It should be noted that in order to prevent denaturation of proteins in the blood sample, it is necessary to set the temperature not to exceed 40°C. However, the higher the temperature of the blood sample, the more easily the erythrocyte aggregation occurs, so that accuracy of the transmitted light measurement can be improved.

The light detection unit 132 may include a light source (irradiation unit) and a light detector (light receiving unit). The light source irradiates the blood sample in the flow path 131 with light. The light source may be implemented by, for example, a near-infrared generator (for example, a near-infrared light emitting diode (LED)). The light detector receives the transmitted light that passes through the blood sample or reflected light (backscattering), from the light that is emitted to the blood sample. The light detector may be implemented by, for example, a photodiode. When the light detector is configured to detect the transmitted light, the light detector is disposed on the opposite side of the light source with the flow path 131 interposed therebetween. When the light detector is configured to detect the reflected light, the light detector is arranged on the same side of the flow path 131 as the light source. The light detection unit 132 detects intensity of received light (hereinafter referred to as "light intensity") from magnitude of the transmitted light or the reflected light with respect to the emitted light, and sends a detection result to the control unit 190. The control unit 190 receives the detection result of the light intensity and stores the detection result in the RAM 193. Hereinafter, a case where intensity of the transmitted light is measured as the light intensity is described as an example.

The control unit 190 functions as an aggregation measurement unit, and measures a degree of erythrocyte aggregation in the blood sample before and after adding the reagent to the blood sample based on the intensity of the transmitted light. More specifically, the aggregation measurement unit calculates a parameter related to the aggregation of the erythrocytes in the blood sample (hereinafter referred to as "aggregation parameter") as the degree of erythrocyte aggregation in the blood sample. Details of the aggregation parameter will be described later. The degree of aggregation can also be represented by a time course (waveform) of the intensity of the transmitted light.

The blood cell count measurement unit 140 measures a blood cell count of the blood sample. The blood cell count measurement unit 140 may include a plurality of measurement units (not shown), and each measurement unit may include a chamber and a blood cell detection unit. The chamber holds the blood sample injected by the nozzle, and the blood cell detection unit measures the blood cell count of the blood sample. The blood sample is injected into the chamber, diluted with the diluent, and hemolyzed with a hemolyzing agent. Then, the blood cell count and the like are measured by the blood cell detection unit. Each chamber is connected to the blood discharge unit 150, and the blood sample after use is discharged to the blood discharge unit 150 by opening each openable and closable electromagnetic valve.

Blood cell count measurement items include, but not limited to, for example, white blood cell count (WBC), red blood cell count (RBC), hemoglobin concentration (HGB), hematocrit value (HCT), mean corpuscular volume (MCV), mean corpuscular hemoglobin content (MCH), mean corpuscular hemoglobin concentration (MCHC), platelet count (PLT), lymphocyte percent (LY%), monocyte percent (MO%), granulocyte percent (GR%), lymphocytes (LY), monocytes (MO), and granulocytes (GR). Among the measurement items, for example, the blood cell count and a blood cell size are measured by an electrical resistance method. HGB is measured based on a colorimetric measurement principle. HCT is measured by a cumulative pulse height method (calculated by RBC histogram) from a blood cell pulse. It should be noted that these blood cell count measurement techniques are all publicly known, and therefore descriptions thereof will be omitted. Blood cell count measurement data is sent to the control unit 190.

The blood discharge unit 150 may include a tube pump 151, the pinch valve 152, a discharge tank (not shown), and the like. The tube pump 151 sucks the blood sample after use from the light measurement unit 130 and discharges the blood sample to the discharge tank in accordance with a command from the control unit 190. The pinch valve 152 opens and closes in accordance with a command from the control unit 190 to control flowing of a fluid from the light measurement unit 130. The discharge tank stores the blood sample after use sucked by the tube pump 151.

The blood sample in the blood storage unit 114 is sucked by the tube pump 151 to be subjected to shear stress, supplied to the flow path 131 through the pipe 119, and flows through the flow path 131 at a predetermined flow rate. The predetermined flow rate is a rate (shear rate) sufficient to release reversible aggregation of the erythrocytes contained in the blood sample. The tube pump 151 and the pinch valve 152 function as a sample supply unit.

The operation input unit 160 is, for example, a touch panel, and receives user instructions, various settings, input data, and the like. The user instructions include, for example, an instruction to start blood type test, an instruction to start measurement of blood cell count, and a setting of forward typing or reverse typing for the blood type test. The input data includes, for example, a mixing ratio of the blood sample and the reagent.

The output unit 170 outputs the measurement data such as the determined blood type and the blood cell count, various setting menus, various operation menus, messages, and the like. Here, the output of the output unit 170 includes, for example, output as a data signal, output of paper on which data is printed, indication on a display, and audio output to a speaker. The output unit 170 may include a connector for sending and receiving data, a printer, a display, a speaker, and the like. The output unit 170 can display both the determined blood type and the measurement result of the blood cell count in accordance with an instruction from the user. In this way, the user can know the determined blood type and the measurement result of the blood cell count at the same time, and the user can quickly obtain information necessary for deciding a treatment for the patient. Details of the output contents of the output unit 170 will be described later.

The power supply unit 180 supplies necessary power to the blood acquisition unit 110, reagent addition unit 120, light measurement unit 130, blood cell count measurement unit 140, blood discharge unit 150, operation input unit 160, output unit 170, and control unit 190.

The control unit 190 functions as a blood type determination unit, and determines the blood type of the blood sample based on a change in the degree of erythrocyte aggregation in the blood sample before and after adding the reagent to the blood sample or after adding the reagent to the blood sample. The blood type determination unit calculates accuracy of the blood type determination and/or agglutination strength of the blood sample based on the aggregation parameter. Methods for calculating the accuracy of the blood type determination and the agglutination strength will be described later.

### <Blood Test Method>

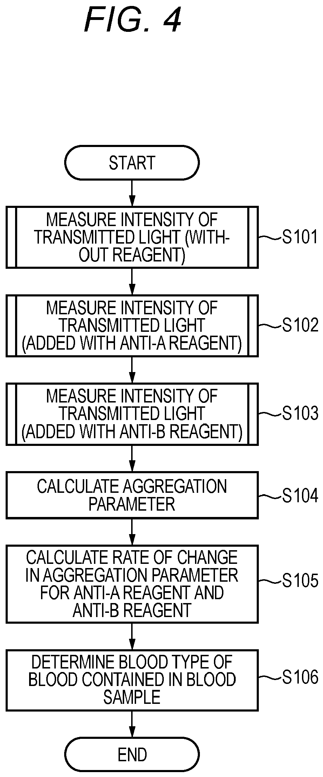

Hereinafter, an outline of a processing procedure of a blood test method by the hemocytometer 100 will be described with reference to FIG. 4. FIG. 4 is a flow chart for explaining the outline of the processing procedure of the blood test method by the hemocytometer 100 according to the present embodiment. The processing of the flow chart in FIG. 4 is implemented by the CPU 191 executing the blood test program.

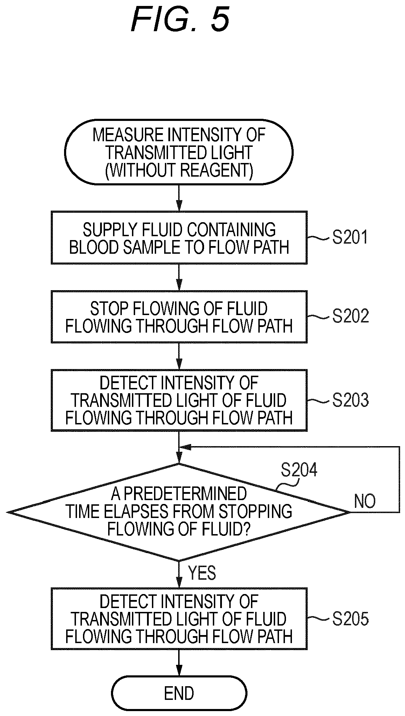

First, intensities of transmitted light (without reagent) is measured (step S101). The light measurement unit 130 measures the intensities of the transmitted light of a fluid containing a blood sample to which no reagent is added. More specifically, the light measurement unit 130 detects the intensities of the transmitted light immediately after flowing of a fluid flowing through the flow path 131 stops and after a predetermined time elapses since the flowing stops, and sends the intensities to the control unit 190. The predetermined time can be in a range of several seconds to ten and several seconds (for example, 10 seconds). A method for measuring the intensity of the transmitted light (without reagent) will be described later with reference to FIG. 5. It should be noted that when a blood sample is collected from a patient in the past and the intensity of the transmitted light (without reagent) is measured, that is, when there is a history of measured intensity of the transmitted light, the intensity of the transmitted light measured in the past can be used. In this way, it is possible to omit the measurement of the intensity of the transmitted light (without reagent), thereby shortening a measurement time.

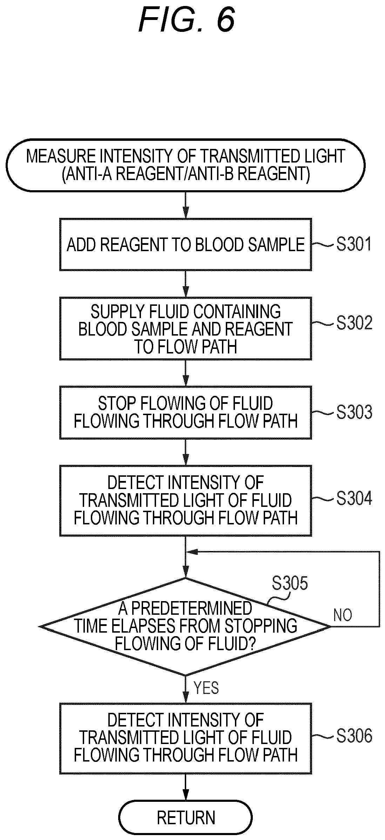

Next, intensities of transmitted light (added with an anti-A reagent) is measured (step S102). The light measurement unit 130 measures the intensities of the transmitted light of a fluid containing a blood sample to which the anti-A reagent is added. More specifically, the light measurement unit 130 detects the intensities of the transmitted light immediately after flowing of a fluid flowing through the flow path 131 stops and after a predetermined time elapses since the flowing stops, and sends the intensities to the control unit 190. A method for measuring the intensity of the transmitted light (added with the anti-A reagent) will be described later with reference to FIG. 6.

Next, intensities of transmitted light (added with an anti-B reagent) is measured (step S103). The light measurement unit 130 measures the intensities of the transmitted light of a fluid containing a blood sample to which the anti-B reagent is added. More specifically, the light measurement unit 130 detects the intensities of the transmitted light immediately after flowing of a fluid flowing through the flow path 131 stops and after a predetermined time elapses since the flowing stops, and sends the intensities to the control unit 190. A method for measuring the intensity of the transmitted light (added with the anti-B reagent) will also be described later with reference to FIG. 6.

Next, the aggregation parameter is calculated (step S104). Based on the intensity of the transmitted light measured by the light measurement unit 130, the aggregation measurement unit calculates the erythrocyte aggregation parameter of the blood sample with no reagent, added with the anti-A reagent, and added with the anti-B reagent. The calculated aggregation parameter with no reagent is referred to ass P0, the aggregation parameter added with the anti-A reagent is referred to as PA, and the aggregation parameter added with the anti-B reagent is referred to as PB. The aggregation parameter can be, for example, AMP. AMP will be described later.

Next, the rate of change of the aggregation parameter is calculated for the anti-A reagent and the anti-B reagent (step S105). The rate of change in the aggregation parameter represents a change in the aggregation parameter of the erythrocytes contained in the blood sample before and after adding the anti-A reagent or the anti-B reagent to the blood sample. For example, the rate of change DA of the aggregation parameter PA when the anti-A reagent is added can be expressed as DA = (PA - P0)/P0 × 100 [%]. The rate of change DB of the aggregation parameter PB when the anti-B reagent is added can be expressed as DB = (PB - P0)/P0 × 100 [%].

It should be noted that the rate of change DA is not limited to the above equation, and the rate of change DA can be calculated by, for example, DA = (PA - P0) PA × 100 [%], DA = PA/(PA + P0) × 100 [%], and DA = P0/(PA + P0) × 100 [%] . Alternatively, it may be configured to calculate DA = PA - P0 or P0 - PA as an amount of change instead of the rate of change. The rate of change DB is also not limited to the above equation, and the rate of change (amount of change) DB can be calculated using other equations in the same manner as the rate of change (or amount of change) DA.

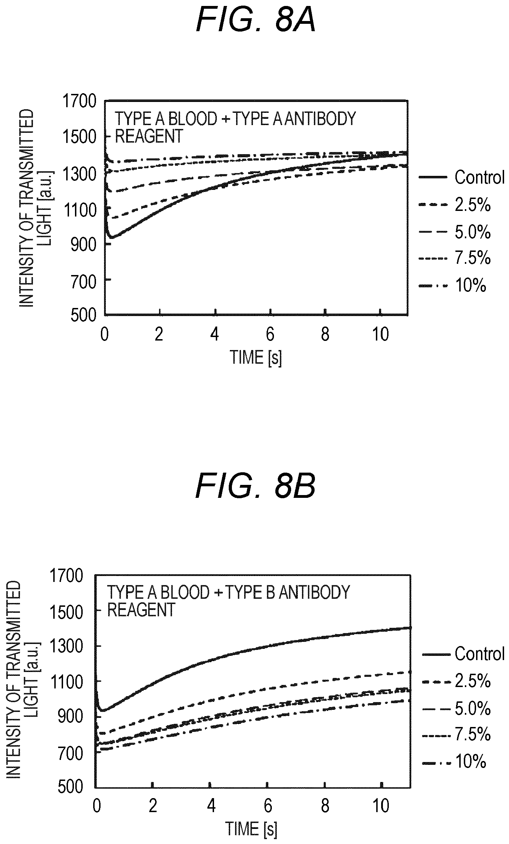

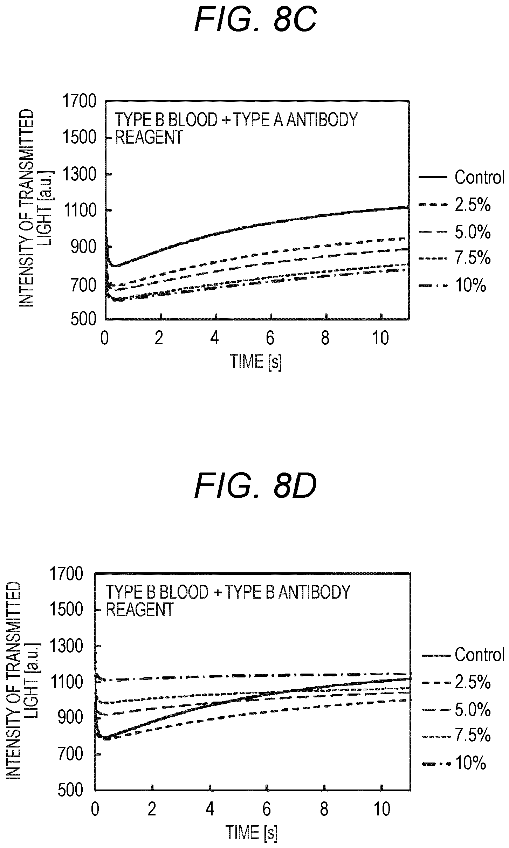

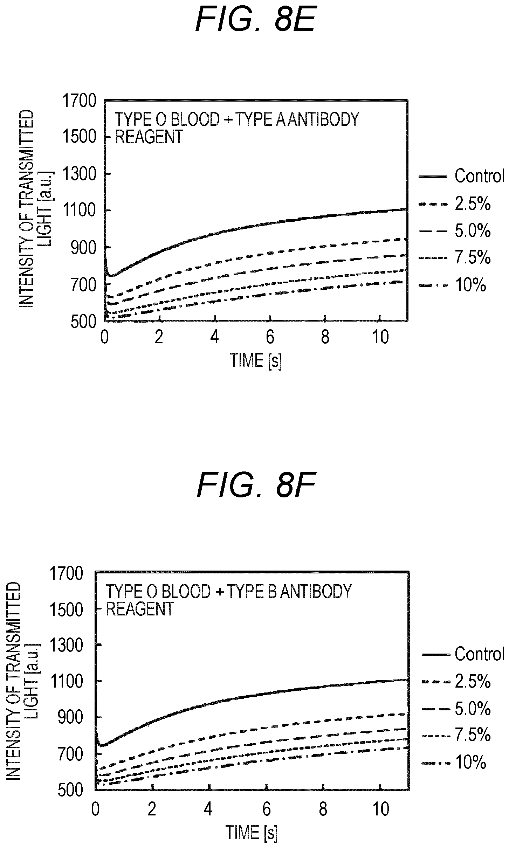

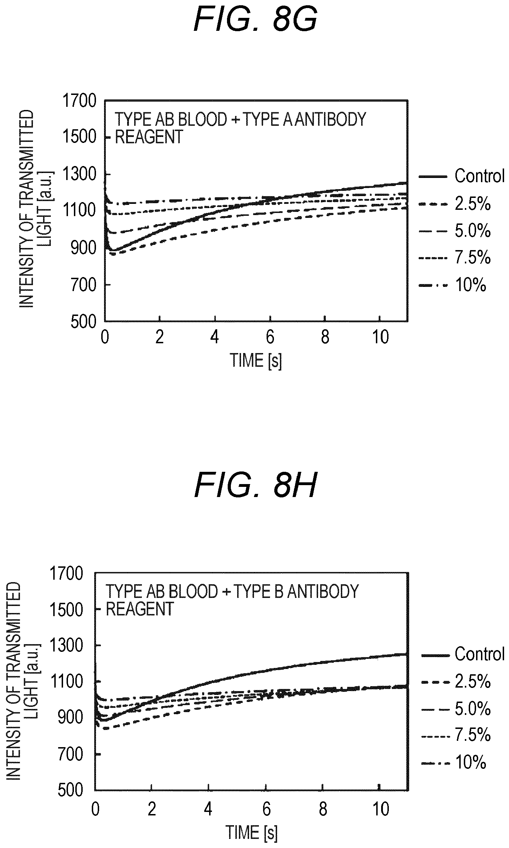

Next, the blood type of the blood sample is determined (step S106). The blood type determining unit determines the blood type of the blood sample based on the rates of change (or amount of change) DA, DB of the aggregation parameter. A method for determining the blood type will be described later with reference to FIGs. 8 to 10.

In this way, in the processing of the flow chart in FIG. 4, the intensity of the transmitted light is measured for each of the cases of no reagent, anti-A reagent addition, and anti-B reagent addition, and the aggregation parameter is calculated based on the intensity of the transmitted light. Subsequently, the rate of change (or amount of change) DA, DB of the aggregation parameter is calculated, and the blood type of the blood sample is determined based on the calculation result.

### <Measurement (S101) of Intensity of Transmitted Light (without Reagent)>

FIG. 5 is a subroutine flow chart for explaining the measurement (S101) processing of the intensity of the transmitted light (without reagent) in the flow chart in FIG. 4. The processing of the subroutine flow chart in FIG. 5 is implemented by the CPU 191 executing the blood test program.

First, the fluid containing the blood sample is supplied to the flow path (step S201). More specifically, the blood sample is sucked from the blood collection tube 111 and held in the nozzle 115, and the electromagnetic valve 117 is opened and the blood sample in the nozzle 115 above the blood storage unit 114 is discharged and injected to the blood storage unit 114 in a predetermined amount. Since no antibody reagent is added to the blood sample in the blood storage unit 114, the erythrocytes contained in the blood sample do not agglutinate due to antigen-antibody reaction, and nonspecifically aggregate over time with a weak force (form rouleaux). It should be noted that when a concentration of the blood sample is high, a diluent can also be supplied to the blood storage unit 114.

The fluid containing the blood sample in the blood storage unit 114 is sucked by tube pump 151 and supplied to the flow path 131 through the pipe 119. In this case, a shear stress is applied to the fluid, and the fluid flows through the flow path 121 at a predetermined flow rate. Due to the flowing of the fluid, the rouleaux of the erythrocytes within the fluid are stirred and thus released.

Subsequently, the flowing of the fluid flowing through the flow path 131 is stopped (step S202). The control unit 190 immediately stops the flowing of the blood sample in the flow path 131 by closing the pinch valve 152. After the flowing of the fluid is stopped, the erythrocytes contained in the blood sample form rouleaux over time.

Subsequently, the intensity of the transmitted light (or backscattering intensity) of the fluid flowing through the flow path 131 is detected (step S203). The light detection unit 132 irradiates the flow path with light from the light source immediately after the flowing of the fluid stops, and receives, by the light detector, transmitted light generated by the light emitted from the light source passing through the flow path 131. The light detection unit 132 detects the intensity of the transmitted light from a magnitude of the transmitted light with respect to the emitted light, and sends a detection result to the control unit 190.

Then, it is determined whether a predetermined time elapses since the flowing of the fluid is stopped (step S204). The predetermined time can be 10 seconds, for example. If the predetermined time elapses (step S204: YES), the light detection unit 132 detects again the intensity of the transmitted light of the fluid flowing through the flow path 131 (step S205), and proceeds to the processing of step S102 in FIG. 4 (return).

### <Measurement (S102/S103) of Intensity of Transmitted Light (Added with Anti-A Reagent/Anti-B Reagent)>

FIG. 6 is a subroutine flow chart for explaining measurement processing (S102/S103) of intensity of transmitted light (added with the anti-A reagent/the anti-B reagent) in the flow chart in FIG. 4. The processing of the subroutine flow chart in FIG. 6 is implemented by the CPU 191 executing the blood test program.

First, the reagent is added to the blood sample (step S301). The reagent addition unit 120 adds the anti-A reagent or the anti-B reagent to the blood sample stored in the blood storage unit 114. When the blood sample in the blood storage unit 114 is type A blood, A antigen in the erythrocytes and anti-A antibody generate specific antigen-antibody reaction, and the erythrocytes agglutinate. When the blood sample in the blood storage unit 114 is type B blood, B antigen in the erythrocytes and anti-B antibody generate specific antigen-antibody reaction, and the erythrocytes agglutinate. When the blood sample in the blood storage unit 114 is type AB blood, the A antigen in the erythrocytes and the anti-A antibody, or the B antigen in the erythrocytes and the anti-B antibody generate the antigen-antibody reaction, and the erythrocytes specifically agglutinate. When the blood sample in the blood storage unit 114 is type O blood, the erythrocytes do not contain the A antigen or the B antigen, so that the erythrocytes do not agglutinate due to the antigen-antibody reaction.

Subsequently, the fluid containing the blood sample and the reagent is supplied to the flow path (step S302). This step is different from the step S201 in that the fluid contains the blood sample and the reagent, but the operation of the sample supply unit in this step is the same as the operation of the sample supply unit in the step S201, so detailed description thereof will be omitted.

Subsequently, the flowing of the fluid flowing through the flow path 131 is stopped (step S303). The control unit 190 immediately stops the flowing of the blood sample in the flow path 131 by closing the pinch valve 152. The agglutination of the erythrocytes due to the antigen-antibody reaction proceeds even after the flowing of the fluid stops. The rouleaux are also formed over time. That is, the agglutination of the erythrocytes in the flow path 131 includes irreversible agglutination due to the antigen-antibody reaction and reversible aggregation (rouleaux).

Subsequently, the intensity of the transmitted light of the fluid flowing through the flow path 131 is detected (step S304). The light detection unit 132 irradiates the flow path with light from the light source immediately after the flowing of the fluid stops, and receives, by the light detector, transmitted light generated by the light emitted from the light source passing through the flow path 131. The light detection unit 132 detects the intensity of the transmitted light from a magnitude of the transmitted light with respect to the emitted light, and sends a detection result to the control unit 190.

Then, it is determined whether a predetermined time elapses since the flowing of the fluid is stopped (step S305). The predetermined time can be 10 seconds, for example. If the predetermined time elapses (step S305: YES), the light detection unit 132 detects again the intensity of the transmitted light of the fluid flowing through the flow path 131 (step S306), and proceeds to the processing of the step S103 in FIG. 4 (when the measurement of the anti-A reagent addition is completed) or S104 (when the measurement of the anti-B reagent addition is completed) (return).

It should be noted that in the processing of the steps S101 to S103, the cases where the intensity of the transmitted light is detected immediately after the flowing of the fluid is stopped and after a predetermined time elapses since the fluid is stopped are described, but the present invention is not limited to such cases. For example, it may be configured to continuously measure the intensity of the transmitted light after starting to supply the fluid to the flow path 131 or after stopping the flowing of the fluid, and acquire the measurement data immediately after stopping the flowing of the fluid and after a predetermined time elapses since the flowing of the fluid is stopped.

### <Method for Calculating Aggregation parameter>

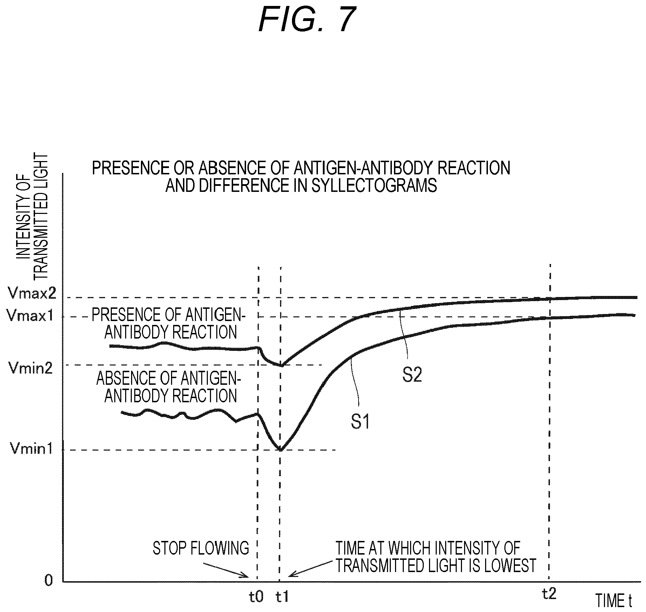

FIG. 7 is a schematic diagram (syllectogram) illustrating a time course of the intensity of the transmitted light before and after the flowing of the blood sample is stopped. In this drawing, a horizontal axis indicates time, and a vertical axis indicates the intensity of the transmitted light. The intensity of the transmitted light corresponds to, for example, an output voltage of a photodiode as the light detector of the light detection unit 132. In the measurement of the syllectogram, the intensity of the transmitted light is continuously measured after starting to supply the fluid to the flow path 131 or after stopping the flowing of the fluid.

In this drawing, S1 represents a syllectogram when no antigen-antibody reaction occurs, and S2 represents a syllectogram when the antigen-antibody reaction occurs. For example, the syllectogram S1 can be a syllectogram when no reagent is added to the blood sample. The syllectogram S2 can be a syllectogram when an antibody reagent of the same type as the blood type of the erythrocytes is added (for example, the anti-A reagent is added to the type A erythrocytes).

In the syllectogram S1, a time at which the flowing of the fluid containing the blood sample flowing through the flow path 131 is stopped is t = t0, and immediately after that time (t = t1), the intensity of the transmitted light reaches a minimum value Vmin1. At the minimum value Vmin1, the light emitted from the light source is scattered and absorbed by the erythrocytes that are individually dissociated and dispersed in the flow path 131, so that the intensity of the transmitted light is minimized. At a time when the flowing is stopped (t = t0), the erythrocytes are deformed and oriented along a flowing direction due to shear stress caused by the flowing, and the amount of the transmitted light is slightly larger than Vmin1.

The intensity of the transmitted light increases after reaching the minimum value Vmin1 at the time t1. This is because when the flowing is stopped, the erythrocytes start to aggregate, and the emitted light passes through gaps between the erythrocytes that are increased by the aggregation. The erythrocytes aggregate since positively charged blood proteins such as fibrinogen, which increase with inflammation, prevent repulsion between negatively charged erythrocytes.

In the syllectogram S2, an antigen-antibody reaction occurs between the antigen of the erythrocytes of the blood sample and the antibody of the reagent (for example, the A antigen of the erythrocytes and the anti-A antibody of the reagent), so that irreversible agglutination of the erythrocytes is formed. Since this irreversible erythrocyte agglutination increases the amount of the transmitted light in the flow path 131, the intensity of the transmitted light of the syllectogram S2 changes with respect to the intensity of the transmitted light of the syllectogram S1 in which no antigen-antibody reaction between the antigen of the erythrocytes of the blood sample and the antibody of the reagent occurs.

For example, in the example shown in FIG. 7, the intensity of the transmitted light of the syllectogram S2 is higher than the intensity of the transmitted light of the syllectogram S1 before and after the flowing of the fluid stops. In addition, from before the flowing of the fluid is stopped to immediately after the flowing of the fluid is stopped, the intensity of the transmitted light of the syllectogram S2 is higher than the intensity of the transmitted light of the syllectogram S1 due to the erythrocyte agglutination generated by the antigen-antibody reaction, but at the time t2, which is a time after a predetermined time elapses from the time t1, a difference in the intensity of the transmitted light therebetween is small. Therefore, a wave height (maximum value - minimum value) of the intensity of the transmitted light of the syllectogram S2 is smaller than a wave height of the light intensity S1. That is, it is considered that the smaller the wave height of the intensity of the transmitted light, the more proceeds the antigen-antibody reaction.

The aggregation measurement unit can calculate the aggregation parameter based on the intensities of the transmitted light (syllectograms S1 and S2). The aggregation parameter can broadly include parameters that correspond to the erythrocyte aggregation. In the present embodiment, a case where a parameter AMP defined by the difference between the maximum value and the minimum value of the intensity of the transmitted light is adopted as the aggregation parameter is exemplified. For example, in order to calculate AMP, the time t2 after a predetermined time (for example, 10 seconds) elapses from the time t1 is set in advance. The predetermined time can be any time at which a rate of increase in the intensity of the transmitted light slows down to some extent and saturates in the syllectogram. At the time t2, the intensity of the transmitted light reaches the maximum value.

For example, the aggregation measurement unit calculates AMP1 for the syllectogram S1, which is a difference between a value of the intensity of the transmitted light (Vmax1) at the time t2 and the minimum value (Vmin1) of the intensity of the transmitted light. The aggregation measurement unit calculates AMP2 for the syllectogram S2, which is a difference between a value of the intensity of the transmitted light (Vmax2) at the time t2 and the minimum value (Vmin2) of the intensity of the transmitted light. A rate of change D in the AMP can be calculated by D = (AMP2 - AMP1)/AMP1 × 100 [%].

It should be noted that although the case of calculating AMP as the aggregation parameter is described, it may be configured to calculate AI instead of AMP. AI is calculated as a ratio of an area of a region B below a curve of the syllectogram in a rectangular region S with a time interval t2 - t1 as one side and AMP as the other side to an area of the region S. That is, the parameter AI is calculated as a ratio of the area of the region B to the sum of an area of a region A and the area of the region B (that is, B/(A+B)) in the syllectogram. The area of the region A is a region above the curve of the syllectogram in the region S.

In addition to AMP and AI, the aggregation parameter can be each value of the area of B, the area of A, the time t1, the minimum value Vmin of the intensity of the transmitted light at the time t1, slope of a rising line of the curve in the syllectogram, and a time t_{1/2}. The slope of the rising line of the curve in the syllectogram is, for example, slope in a linear increase section of a limited time period from the time t1 to t2 of the syllectogram in FIG. 7. The time t_{1/2} is a time when the intensity of the transmitted light increases from Vmin by AMP/2.

### <Method for Determining Blood Type>

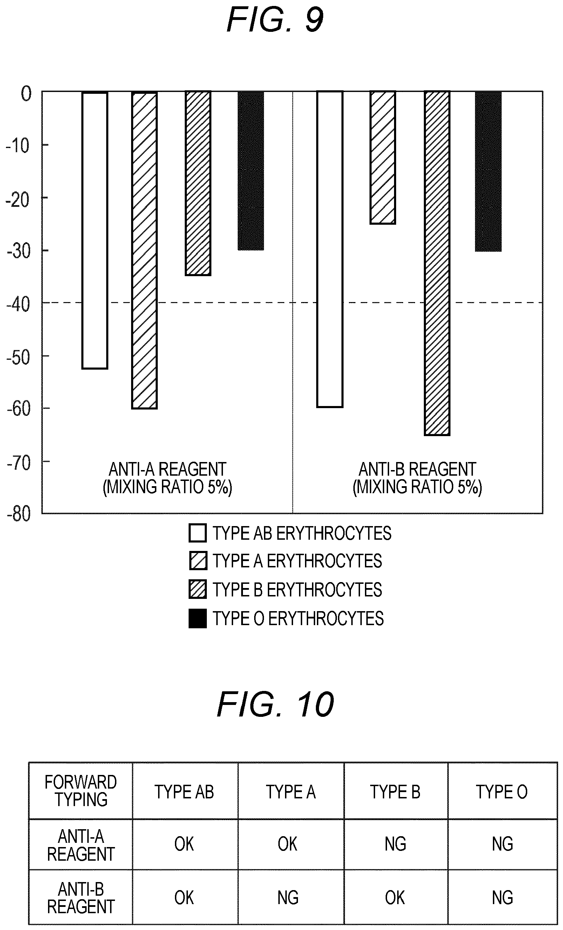

FIGs. 8A to 8H are graphs illustrating measurement results of the intensity of the transmitted light for blood samples of different blood types after the flowing of the fluid is stopped. In the graphs, a vertical axis indicates the intensity of the transmitted light [a.u.] and a horizontal axis indicates time [s]. Solid lines in the graphs indicate measurement results when no reagent is added ("Control"), and dashed lines and alternate long and short dash lines indicate measurement results when mixing ratios of the blood sample and the reagent are 2.5, 5.0, 7.5, and 10 [%]. FIG. 9 is a graph illustrating calculation results of the rate of change in the aggregation parameter (AMP) before and after addition of the reagent (with a mixing ratio of 5%), and FIG. 10 is a diagram illustrating a relation between the antigen-antibody reaction and the blood type in the forward typing.

FIG. 8A shows measurement results of the intensity of the transmitted light from the flowing of the fluid flowing through the flow path 131 is stopped until approximately 10 seconds elapses in the case where the anti-A reagent is added to the blood sample containing type A erythrocytes. Immediately after the flowing of the fluid stops (from before stopping), the intensity of the transmitted light when the anti-A reagent is added is higher than the intensity of the transmitted light when the anti-A reagent is not added (control). This is because when the anti-A reagent is added, the erythrocyte agglutination due to the antigen-antibody reaction proceeds before the flowing of the fluid is stopped, and even after the flowing of the fluid is stopped, the erythrocyte agglutination due to the antigen-antibody reaction and the rouleaux formation further proceed. Then, the difference between the intensity of the transmitted light when the anti-A reagent is added and the intensity of the transmitted light of the control decreases with the passage of time after stopping the flowing of the fluid, but the wave height of the intensity of the transmitted light when the anti-A reagent is added is smaller than the wave height of the intensity of the transmitted light of the control. The higher the mixing ratio, the higher the intensity of the transmitted light from before to after the flowing of the fluid is stopped, and the faster the rate of agglutination due to the antigen-antibody reaction.

FIG. 8B shows measurement results of the intensity of the transmitted light from the flowing of the fluid flowing through the flow path 131 is stopped until approximately 10 seconds elapses in the case where the anti-B reagent is added to the blood sample containing type A erythrocytes. After the flowing of the fluid stops, the intensity of the transmitted light when the anti-B reagent is added is lower than the intensity of the transmitted light when the anti-B reagent is not added (control). This is because when the anti-B reagent is added, the antigen-antibody reaction does not occur and the rouleaux formation of the erythrocytes proceeds after the flowing of the fluid is stopped, but due to the influence of electrolytes and albumin contained in the anti-B reagent, the erythrocyte aggregation is inhibited and light transmission is reduced.

FIG. 8C shows measurement results of the intensity of the transmitted light from the flowing of the fluid flowing through the flow path 131 is stopped until approximately 10 seconds elapses in the case where the anti-A reagent is added to the blood sample containing type B erythrocytes. After the flowing of the fluid stops, the intensity of the transmitted light when the anti-A reagent is added is lower than the intensity of the transmitted light when the anti-A reagent is not added (control). This is because when the anti-A reagent is added, the antigen-antibody reaction does not occur and the rouleaux formation of the erythrocytes proceeds after the flowing of the fluid is stopped, but due to the influence of electrolytes and albumin contained in the anti-A reagent, the erythrocyte aggregation is inhibited and light transmission is reduced.

FIG. 8D shows measurement results of the intensity of the transmitted light from the flowing of the fluid flowing through the flow path 131 is stopped until approximately 10 seconds elapses in the case where the anti-B reagent is added to the blood sample containing type B erythrocytes. Immediately after the flowing of the fluid stops (from before stopping), the intensity of the transmitted light when the anti-B reagent is added is higher than the intensity of the transmitted light when the anti-B reagent is not added (control). This is because when the anti-B reagent is added, the erythrocyte agglutination due to the antigen-antibody reaction proceeds before the flowing of the fluid is stopped, and even after the flowing of the fluid is stopped, the erythrocyte agglutination due to the antigen-antibody reaction and the rouleaux formation further proceed. The wave height of the intensity of the transmitted light when the anti-B reagent is added is smaller than the wave height of the intensity of the transmitted light of the control. The higher the mixing ratio, the higher the intensity of the transmitted light from before to after the flowing of the fluid is stopped, and the faster the rate of agglutination due to the antigen-antibody reaction.

FIG. 8E shows measurement results of the intensity of the transmitted light from the flowing of the fluid flowing through the flow path 131 is stopped until approximately 10 seconds elapses in the case where the anti-A reagent is added to the blood sample containing type O erythrocytes. After the flowing of the fluid stops, the intensity of the transmitted light when the anti-A reagent is added is lower than the intensity of the transmitted light when the anti-A reagent is not added (control). This is because when the anti-A reagent is added, the antigen-antibody reaction does not occur and the rouleaux formation of the erythrocytes proceeds after the flowing of the fluid is stopped, but due to the influence of electrolytes and albumin contained in the anti-A reagent, the erythrocyte aggregation is inhibited and light transmission is reduced.

FIG. 8F shows measurement results of the intensity of the transmitted light from the flowing of the fluid flowing through the flow path 131 is stopped until approximately 10 seconds elapses in the case where the anti-B reagent is added to the blood sample containing type O erythrocytes. After the flowing of the fluid stops, the intensity of the transmitted light when the anti-B reagent is added is lower than the intensity of the transmitted light when the anti-B reagent is not added (control). This is because when the anti-B reagent is added, the antigen-antibody reaction does not occur and the rouleaux formation of the erythrocytes proceeds after the flowing of the fluid is stopped, but due to the influence of electrolytes and albumin contained in the anti-B reagent, the erythrocyte aggregation is inhibited and light transmission is reduced.

FIG. 8G shows measurement results of the intensity of the transmitted light from the flowing of the fluid flowing through the flow path 131 is stopped until approximately 10 seconds elapses in the case where the anti-A reagent is added to the blood sample containing type AB erythrocytes. Immediately after the flowing of the fluid stops (from before stopping), the intensity of the transmitted light when the anti-A reagent is added is higher than the intensity of the transmitted light when the anti-A reagent is not added (control), except when the mixing ratio is 2.5 [%]. This is because when the anti-A reagent is added, the erythrocyte agglutination due to the antigen-antibody reaction proceeds before the flowing of the fluid is stopped, and even after the flowing of the fluid is stopped, the erythrocyte agglutination due to the antigen-antibody reaction and the rouleaux formation further proceed. The wave height of the intensity of the transmitted light when the anti-A reagent is added is smaller than the wave height of the intensity of the transmitted light of the control. The higher the mixing ratio, the higher the intensity of the transmitted light from before to after the flowing of the fluid is stopped, and the faster the rate of agglutination due to the antigen-antibody reaction.

FIG. 8H shows measurement results of the intensity of the transmitted light from the flowing of the fluid flowing through the flow path 131 is stopped until approximately 10 seconds elapses in the case where the anti-B reagent is added to the blood sample containing type AB erythrocytes. Immediately after the flowing of the fluid stops (from before stopping), the intensity of the transmitted light when the anti-B reagent is added is higher than the intensity of the transmitted light when the anti-B reagent is not added (control), except when the mixing ratio is 2.5 [%]. This is because when the anti-A reagent is added, the erythrocyte agglutination due to the antigen-antibody reaction proceeds before the flowing of the fluid is stopped, and even after the flowing of the fluid is stopped, the erythrocyte agglutination due to the antigen-antibody reaction and the rouleaux formation further proceed. Then, the difference between the intensity of the transmitted light when the anti-A reagent is added and the intensity of the transmitted light of the control increases with the passage of time after stopping the flowing of the fluid, but the wave height of the intensity of the transmitted light when the anti-B reagent is added is smaller than the wave height of the intensity of the transmitted light of the control. The higher the mixing ratio, the higher the intensity of the transmitted light from before to after the flowing of the fluid is stopped, and the faster the rate of agglutination due to the antigen-antibody reaction.

Therefore, by comparing a waveform of the intensity of the transmitted light when the reagent is added to the blood sample (syllectogram S2) and a waveform of the intensity of the transmitted light of the control (syllectogram S1), presence or absence of erythrocyte agglutination due to the antigen-antibody reaction when the reagent is added can be estimated. More specifically, the blood type determination unit determines which waveform pattern among the above-described FIGs. 8A to 8H corresponds to the syllectograms S1 and S2, so as to estimate the presence or absence of erythrocyte agglutination due to the antigen-antibody reaction when the reagent is added to the blood sample.

Correspondence between the syllectograms S1 and S2 and the waveform patterns of FIGs. 8A to 8H can be performed by, for example, comparing statistical information (or feature amounts) of syllectograms S1 and S2 and statistical information (or feature amounts) of the waveform patterns of FIGs. 8A to 8H, and then determining a degree of similarity between the two.

It may be configured to input waveform data of the syllectograms S1 and S2 or image data of the waveforms as input data to a learned classification model implemented by a neural network, and classify the input data by the classification model, so as to estimate the blood type as a type of input data. A machine learning device that machine-learns the classification model inputs a plurality of pieces of the input data and correct data of the blood types corresponding to the input data into a learning model, and performs machine learning on the learning model with the correct data as a target, so as to generate the learned model. For example, thousands to tens of thousands of sets of the input data and the correct data are prepared and stored in advance in the auxiliary memory 194. The generated learned model is stored in the auxiliary memory 194.

Then, the blood type determination unit estimates the presence or absence of erythrocyte agglutination due to the antigen-antibody reaction in the cases where the anti-A reagent and the anti-B reagent are added to the blood sample, respectively, so as to estimate the blood type of the blood sample from the relation between the antigen-antibody reaction and the blood type in the forward typing (see FIG. 10).

In this way, the blood type of the blood sample can be determined based on the change (waveform) in the time course of the intensity of the transmitted light before and after the reagent is added to the blood sample from the syllectograms S1 and S2 measured for the blood sample. Here, the time course of the intensity of the transmitted light represents a degree of the erythrocyte aggregation.

It should be noted that when a blood sample is collected in the past for a patient and the syllectogram S1 is measured, that is, when there is a measurement history of the syllectogram S1, the most recent syllectogram S2 of the same patient and the syllectogram S1 measured in the past can also be used to determine the blood type of the blood sample. In this way, it is possible to omit the measurement of the syllectogram S1, thereby shortening the measurement time.

The blood type of the blood sample can also be determined based on the change (waveform) in the time course of the intensity of the transmitted light of the blood sample added with a reagent using the syllectogram S2 but not using the syllectogram S1.

For example, in FIGs. 8A to 8H, the intensities of the transmitted light of the syllectograms S2 and slopes thereof are different from each other depending on the combination of the blood type and the reagent and the mixing ratio. For example, when comparing "type A blood + type A antibody reagent" and "type A blood + type B antibody reagent", the intensity of the transmitted light of "type A blood + type A antibody reagent" is higher regardless of the mixing ratio and the time that elapses. The slope of the intensity of the transmitted light of "type A blood + type A antibody reagent" is smaller. On the other hand, when comparing "type B blood + type A antibody reagent" and "type B blood + type B antibody reagent", the intensity of the transmitted light of "type B blood + type B antibody reagent" is higher when the mixing ratio is 5.0 [%] or more, regardless of the time that elapses. The slope of the intensity of the transmitted light of "type B blood + type B antibody reagent" is smaller.

The slope of the intensity of the transmitted light in "type O blood + type A antibody reagent" and "type O blood + type B antibody reagent" is larger than the slope of the intensity of the transmitted light in "type A blood + type A antibody reagent" and "type B blood + type B antibody reagent". On the other hand, the slope of the intensity of the transmitted light in "type AB blood + type A antibody reagent" and "type AB blood + type B antibody reagent" is the same as the slope of the intensity of the transmitted light in "type A blood + type A antibody reagent" and "type B blood + type B antibody reagent".

In this way, the waveform of the syllectogram S2 changes depending on whether the antigen-antibody reaction occurs due to the combination of the blood type and the reagent. For example, when the slope of the intensity of the transmitted light is smaller than a predetermined first threshold value, the blood type determination unit determines that the antigen-antibody reaction occurs, and when the slope of the intensity of the transmitted light is larger than the first threshold value, the blood type determination unit determines that no antigen-antibody reaction occurs. The first threshold value can be set based on, for example, results obtained by conducting an experiment for a plurality of blood samples to determine whether the antigen-antibody reaction occurs. The blood type determination unit determines whether the antigen-antibody reaction occurs from the slope of the intensity of the transmitted light, and estimates the blood type of the blood sample from the relation between the antigen-antibody reaction and the blood type in the forward typing (see FIG. 10).

It may be configured to input waveform data of the syllectogram S2 or image data of the waveform as input data to a learned classification model implemented by a neural network, and classify the input data by the classification model, so as to estimate the blood type as a type of input data. A machine learning device that machine-learns the classification model inputs a plurality of pieces of the input data and correct data of the blood types corresponding to the input data into a learning model, and performs machine learning on the learning model with the correct data as a target, so as to generate the learned model. For example, thousands to tens of thousands of sets of the input data and the correct data are prepared and stored in advance in the auxiliary memory 194. The generated learned model is stored in the auxiliary memory 194.

By evaluating a state of proceeding of the erythrocyte agglutination due to the antigen-antibody reaction with respect to a state of proceeding of the reversible erythrocyte aggregation with the rate of change D in AMP, the blood type can be determined taking into account the state of proceeding of the erythrocyte agglutination due to the antigen-antibody reaction. In this way, it is possible to improve the accuracy of blood type determination.

In the rate of change D in AMP, the wave height (AMP1) of the intensity of the transmitted light of the control represents an increase in the reversible erythrocyte aggregation. The wave height (AMP2) of the intensity of the transmitted light when the reagent is added represents an increase in the reversible erythrocyte aggregation obtained by subtracting the increase in the erythrocyte agglutination due to the antigen-antibody reaction from the increase in the erythrocyte aggregation of the control. That is, when the flowing of the fluid stops, the AMP2 value decreases as the erythrocyte agglutination due to the antigen-antibody reaction proceeds. Therefore, as the erythrocyte agglutination due to the antigen-antibody reaction proceeds, a value of (AMP2 - AMP1) decreases (negatively increases). Furthermore, by dividing (AMP2 - AMP1) by AMP1 and matching the scales between the blood samples, comparison between different blood samples is possible.

It should be noted that there is a possibility that the smaller the AMP1 value, the less proceeds the reversible erythrocyte aggregation in the control, and the more errors there are in measuring the aggregation parameter (AMP). When AMP1 is smaller than a predetermined second threshold value, the blood type determination unit determines that an error occurs during the measurement of the aggregation parameter (AMP). The second threshold value can be experimentally set from the viewpoint of whether an error occurs during the AMP measurement.

As shown in FIG. 9, when the anti-A reagent is added to each blood sample containing AB type, A type, B type, or O type erythrocytes, the erythrocyte agglutination due to the antigen-antibody reaction occurs in the AB type and A type blood samples, whereas the erythrocyte agglutination due to the antigen-antibody reaction does not occur in the B type and O type blood samples. As a result, the rate of change in AMP of the AB type and A type blood samples is lower than the rate of change in AMP of the B type and O type blood samples (an absolute value of the rate of change is greater).

When the anti-B reagent is added to each blood sample containing AB type, A type, B type, or O type erythrocytes, the erythrocyte agglutination due to the antigen-antibody reaction occurs in the AB type and B type blood samples, whereas the erythrocyte agglutination due to the antigen-antibody reaction does not occur in the A type and O type blood samples. As a result, the rate of change in AMP of the AB type and B type blood samples is lower than the rate of change in AMP of the A type and O type blood samples (an absolute value of the rate of change is greater).

The blood type determination unit can distinguish whether the erythrocyte agglutination due to the antigen-antibody reaction occurs depending on whether the absolute value of the rate of change in AMP is equal to or less than an absolute value of a threshold value (a third threshold value) or exceeds the third threshold value. When the rate of change in AMP is equal to or less than the third threshold value, it is determined that the erythrocyte agglutination due to the antigen-antibody reaction occurs, and when the absolute value of the rate of change in AMP exceeds the absolute value of the third threshold value, it is determined that the erythrocyte agglutination due to the antibody reaction does not occur. The third threshold value can be experimentally set according to the mixing ratio of the reagent. In the example shown in FIG. 9, for example, by setting the third threshold value to - 40 [%], it is possible to distinguish between blood types in which the erythrocyte agglutination due to the antigen-antibody reaction occurs and blood types in which it does not. That is, as shown in FIG. 9, when the rate of change in AMP is - 40 [%] or less, it can be determined that the erythrocyte agglutination due to the antigen-antibody reaction occurs between the antigen in the blood sample and the antibody in the reagent.

As shown in FIG. 10, from a correspondence between a combination of erythrocyte agglutination due to antigen-antibody reaction ("o" in the drawing) and no erythrocyte agglutination due to antigen-antibody reaction ("×" in the drawing) and blood types, the blood type can be determined based on the presence or absence of the erythrocyte agglutination due to the antigen-antibody reaction with respect to the anti-A reagent and the anti-B reagent in the blood sample. For example, when the erythrocyte agglutination due to the antigen-antibody reaction occurs for both the anti-A reagent and the anti-B reagent, the blood type determination unit determines the blood sample as "AB type".

The blood type determination unit can also estimate the accuracy of blood type determination according to the value of the rate of change in AMP. For example, when the rate of change in AMP (for example, an average rate of change in AMP for the blood types with the erythrocyte agglutination due to the antigen-antibody reaction) is less than - 50 [%], the accuracy of blood type determination can be estimated to be "high", and when the rate of change in AMP is - 50 [%] or more, the accuracy of blood type determination can be estimated to be "low". Furthermore, it is not limited to estimating the accuracy of blood type determination as "high" and "low", and it is also possible to estimate the accuracy of blood type determination in a plurality of stages according to the value of the rate of change in AMP, and represent the accuracy by a level (a numerical value).

Furthermore, the blood type determination unit can also estimate the agglutination strength according to the value of the rate of change in AMP. For example, when the rate of change in AMP is less than - 50 [%], the agglutination strength is estimated to be "strong", and when the rate of change in AMP is - 50 [%] or more, the agglutination strength is estimated to be "weak". Furthermore, it is not limited to estimating the agglutination strength as "strong" and "weak", and it is also possible to estimate the agglutination strength in a plurality of stages according to the value of the rate of change in AMP, and represent the agglutination strength by a level (a numerical value).

### <Output Method>

In addition to the blood type determined by the blood type determination unit, the output unit 170 can output at least one of the accuracy of blood type determination, the aggregation parameter of the erythrocytes contained in the blood sample, the agglutination strength, and the error in measuring the aggregation parameter.

The accuracy of blood type determination can be output as, for example, "high" or "low" or a level (numerical value). In this way, the user can know the determination accuracy of the determined blood type.

The agglutination strength can be output as, for example, "strong" or "weak" or a level (numerical value). In this way, the user can know the agglutination strength.

The aggregation parameter can be output as, for example, the AMP value [%] or a score value corresponding to the AMP value, for the case where the reagent is not added (control) and the case where the reagent is added. The error in measuring the aggregation parameter can be output as presence or absence of error. In this way, the user can know whether the measured aggregation parameter is a valid value or whether it is correct.

### <Main Effects>

The hemocytometer 100 (blood test apparatus), blood test method, and blood test program according to the present embodiment described above have the following effects.

The degree of erythrocyte aggregation of the blood sample before and after adding the reagent containing the blood type antibody or the blood type antigen to the blood sample (the time course of the intensity of the transmitted light or the aggregation parameter) is measured, and the blood type of the blood sample is determined based on the change in the degree of erythrocyte aggregation before and after the addition of the reagent. Therefore, it is possible to distinguish the agglutinations due to the antigen-antibody reaction from the rouleaux, so that the blood type can be determined with high accuracy.

In the methods in the related art of visually confirming the presence or absence of the erythrocyte agglutination, such as the test tube method and the slide method, a sufficient amount of reagent is required for the agglutination reaction to produce clumps of a visually distinguishable size. For example, in the slide method and the like, a ratio of the blood sample and the reagent can be 1:1, that is, the mixing ratio can be approximately 50%. In contrast, the hemocytometer 100 according to the present embodiment uses an extremely small amount of reagent, and measures the degree of erythrocyte aggregation by absorptiometry, so that even a slight change in aggregation can also be detected. Therefore, the hemocytometer 100 can determine the blood type using a much smaller amount of the reagent than the methods in the related art, and thus is economical. The measurement time for each type of the anti-A reagent and the anti-B reagent is merely approximately several seconds to approximately 10 seconds, and short-time measurement is possible. In addition, unlike the column agglutination method, it does not require a complicated cartridge, so that the blood type determination can be implemented at a low cost. As described above, the hemocytometer 100 can omit the steps of cleaning the erythrocytes and diluting the blood sample, and can reduce the amount of the reagent, thereby shortening the time and reducing the cost of the blood type test process.

### (Second Embodiment)

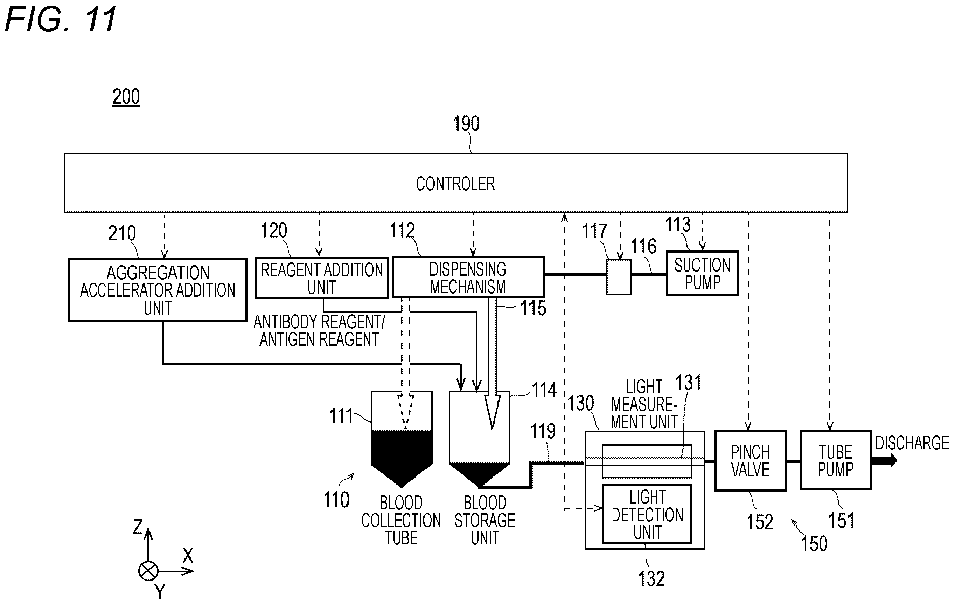

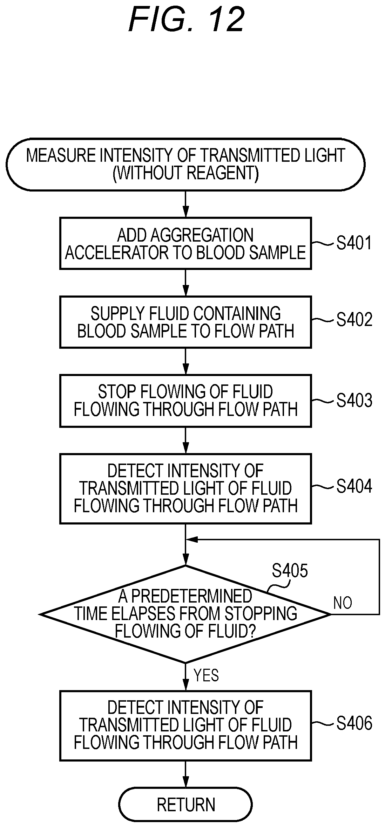

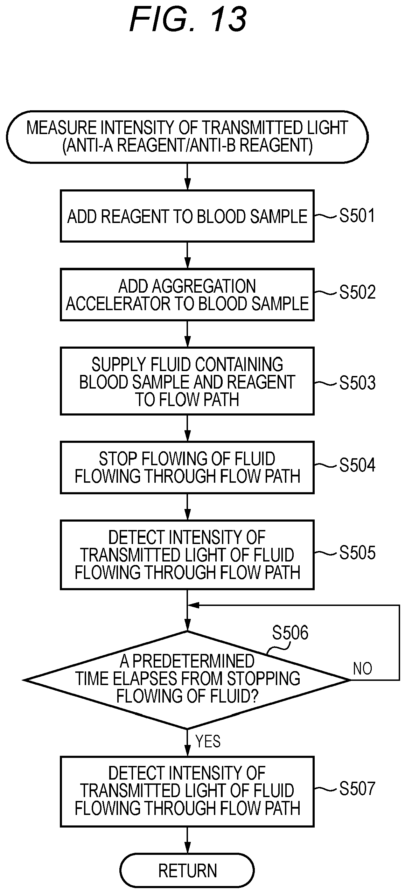

FIG. 11 is a schematic diagram illustrating a schematic configuration of a hemocytometer 200 according to a second embodiment, and FIG. 12 is a subroutine flow chart for explaining measurement processing (S101) of the intensity of the transmitted light according to the second embodiment. FIG. 13 is a subroutine flow chart for explaining measurement processing (S102/S103) of the intensity of the transmitted light according to the second embodiment. The processing of the subroutine flow charts in FIGs. 12 and 13 is implemented by the CPU 191 executing the blood test program.

In the present embodiment, a case where a substance that promotes the rouleaux reaction (reversible erythrocyte aggregation) of the erythrocytes (hereinafter referred to as "aggregation-accelerating substance" in this description) is added so that changes in the syllectograms and the aggregation parameter before and after the antigen-antibody reaction become large will be described. It should be noted that in the following description, detailed descriptions of the same configurations as those of the first embodiment will be omitted in order to avoid duplication of description.

As shown in FIG. 11, the hemocytometer 200 according to the present embodiment may further include an agglutination accelerator addition unit 210 in addition to the configurations of the hemocytometer 100 according to the first embodiment described above.

The agglutination accelerator addition unit 210 adds the aggregation-accelerating substance to the blood sample stored in the blood storage unit 114. In the blood storage unit 114, since the erythrocytes of the blood sample before the addition of the aggregation-accelerating substance are negatively charged, a repulsive force acts between the erythrocytes. On the other hand, various proteins and macromolecular substances are known to cross-link the erythrocytes to promote the aggregation of erythrocytes. By adding these proteins and macromolecular substances to the blood sample as the aggregation-accelerating substance, the erythrocyte aggregation is accelerated when the flowing of the fluid is stopped.

Examples of the aggregation-accelerating substance include proteins in vivo such as fibrinogen and immunoglobulin, and various macromolecular substances such as saccharides, such as macromolecular dextran, polyethylene glycol, gelatin, starch, and polyvinylpyrrolidone.

As described in the above-described first embodiment, the blood type determination unit determines the blood type of the blood sample based on the change in the degree of erythrocyte aggregation in the blood sample before and after adding the reagent to the blood sample. In such a blood type determination method, when the degree of erythrocyte aggregation is low, that is, when the reversible erythrocyte aggregability is low in the case of not adding any antibody reagent (control), the change in the degree of erythrocyte aggregation in the blood sample before and after the addition of the antibody reagent may be small, and the accuracy of blood type determination may decrease. In order to avoid such a decrease in the determination accuracy, by adding an equal amount of a reagent containing the aggregation-accelerating substance (hereinafter referred to as an "aggregation accelerator") during the measurement of the intensity of the transmitted light of the control and the measurement of the intensity of the transmitted light when the antibody reagent is added, it is possible to enhance a difference in transition of the intensity of the transmitted light before and after the addition of the antibody reagent. The aggregation accelerator is added to the dispensed blood sample in the blood storage unit 114 in accordance with a command from the control unit 190 in the same manner as the antibody reagent. Alternatively, an antibody reagent containing the aggregation accelerator in advance may be added to the blood sample in the blood storage unit 114.

As shown in FIG. 12, in the present embodiment, in the measurement of the intensity of the transmitted light when no reagent is added, before the fluid containing the blood sample is supplied to the flow path 131, the aggregation accelerator is added to the blood sample stored in the blood storage unit 114 (S401). The subsequent processing (steps S402 to S406) in the same drawing are the same as the processing in the steps S201 to S205 in the first embodiment, so that detailed description thereof will be omitted.

As shown in FIG. 13, in the measurement of the intensity of the transmitted light when the reagent is added, after the reagent is added to the blood sample and before the fluid containing the blood sample is supplied to the flow path 131, the agglutination accelerator is added to the blood sample stored in the blood storage unit 114 (S502). The processing of adding the reagent to the blood sample (step S501) and the processing of steps S503 to S507 in the drawing are the same as the processing of the steps S301 to S306 in the first embodiment, so that detailed description thereof will be omitted.

In this way, in the present embodiment, the intensity of the transmitted light is measured after adding the same amount of the aggregation accelerator to the blood sample when no reagent is added and when the reagent is added. Therefore, the reversible erythrocyte aggregation of the erythrocytes is accelerated, and the change in the degree of erythrocyte aggregation of the blood sample before and after the addition of the antibody reagent (that is, the change in the syllectograms and the aggregation parameter) becomes large. Therefore, the accuracy of blood type determination can be well maintained.

Although the blood test apparatus, blood test method, and blood test program according to the embodiments of the presently disclosed subject matter are described above, the presently disclosed subject matter is not limited to the above-described embodiments.

For example, by containing a plurality of reagents in disposable cartridges, respectively, and sucking the blood sample into the cartridges, the blood sample and the reagent may be automatically mixed for measurement.

Although the determination of the ABO blood types is mainly explained in the above-described embodiments, as for the Rh blood type, erythrocytes are used in the same manner as in the forward typing to confirm presence or absence of irreversible erythrocyte agglutination due to the antigen-antibody reaction with anti-D antibody, so as to determine Rh-positive or Rh-negative.

Although the case where the hemocytometer 100 automatically performs from the measurement of the intensity of the transmitted light to the determination of the blood type is described in the above-described embodiments, but the present invention is not limited to such a case, and some steps may be manually performed by the user. For example, the user may specify the procedure of a plurality of steps included in the measurement of the intensity of the transmitted light when no reagent is added to the blood sample and the measurement of the intensity of the transmitted light when the reagent is added to the blood sample. In the above examples, the case where the reagent is automatically added to the blood sample by the reagent addition unit 120 is described, but the user may manually select the reagent to be added and specify the amount to be added.

Further, after the measurement of the intensity of the transmitted light in the case where no reagent is added to the blood sample is completed, the fluid containing the blood sample used for the measurement may be divided into half without being discarded, then added with the anti-A reagent and the anti-B reagent respectively, and then sequentially supplied to the flow path 131 to measure the intensity of the transmitted light. Alternatively, a plurality of blood storage units 114 and a plurality of light measurement units 130 may be provided, so that the measurement of the intensity of the transmitted light when no reagent is added to the blood sample and the measurement of the intensity of the transmitted light when the reagent is added to the blood sample may be performed at the same time in the plurality of light measurement units 130. As a result, the measurement time can be shortened compared with the case of sequentially measuring the intensities of the transmitted light when no reagent is added to the blood sample and when the reagent is added.

In the above-described embodiments, the blood test apparatus is described by exemplifying the hemocytometer 100 including the blood cell count measurement unit 140, but the blood testing apparatus may also be configured as an apparatus not including the blood cell count measurement unit 140.

A part or all of the functions executed by the blood test program in the above embodiments may be executed by hardware such as an electronic circuit.

## Claims

1. A blood test apparatus, comprising:
a blood acquisition unit that acquires a blood sample;
a reagent addition unit that adds a reagent to the blood sample;
an aggregation measurement unit that measures a degree of erythrocyte aggregation of the blood sample added with the reagent; and
a blood type determination unit that determines a blood type of the blood sample based on a change in the degree of erythrocyte aggregation of the blood sample added with the reagent, wherein
the reagent contains an antibody that generates antigen-antibody reaction with an antigen contained in the blood sample, or an antigen that generates antigen-antibody reaction with an antibody contained in the blood sample.

2. The blood test apparatus according to claim 1, wherein
the aggregation measurement unit measures the degree of erythrocyte aggregation of the blood sample before and after adding the reagent to the blood sample, and
the blood type determination unit determines the blood type of the blood sample based on a change in the degree of erythrocyte aggregation of the blood sample before and after adding the reagent to the blood sample.

3. The blood test apparatus according to claim 1 or 2, wherein
the aggregation measurement unit includes
a flow path that allows the blood sample to flow, and
a light detection unit that includes an irradiation unit irradiating the flow path with light and a light receiving unit receiving light generated by the light emitted from the irradiation unit passing through or reflected by the flow path, and
the degree of erythrocyte aggregation of the blood sample is measured based on intensity of the light received by the light receiving unit.

4. The blood test apparatus according to claim 3, further comprising:
a sample supply unit that supplies the blood sample to the flow path; and
a control unit that controls the sample supply unit so as to allow the blood sample to flow at a predetermined flow rate at which reversible aggregation of erythrocytes contained in the blood sample is released and then to stop the flowing of the blood sample before and after adding the reagent to the blood sample, wherein
the reagent is a blood type antibody reagent containing an antibody that generates antigen-antibody reaction with an antigen in the erythrocytes, and
the aggregation measurement unit measures the degree of erythrocyte aggregationof the blood sample based on a time course of the intensity of the light after the flowing of the blood sample is stopped.

5. The blood test apparatus according to claim 4, wherein
the aggregation measurement unit measures the degree of erythrocyte aggregationof the blood sample based on a difference between a maximum value and a minimum value of the intensity of the light after the flowing of the blood sample is stopped.

6. The blood test apparatus according to any one of claims 1 to 3, wherein
the reagent addition unit adds to the blood sample a blood type antibody reagent that generates antigen-antibody reaction with an antigen in the erythrocytes contained in the blood sample.

7. The blood test apparatus according to any one of claims 1 to 3, wherein
the reagent addition unit adds to the blood sample a blood type antigen reagent that generates antigen-antibody reaction with an antibody in blood plasma contained in the blood sample.

8. The blood test apparatus according to any one of claims 1 to 7, further comprising:
an output unit that outputs at least one of the blood type determined by the blood type determination unit, accuracy of the blood type determination, the degree of erythrocyte aggregation of the blood sample, and an error during measurement of the degree of erythrocyte aggregation.

9. The blood test apparatus according to claim 8, further comprising:
a blood cell count measurement unit that measures a blood cell count of the blood sample, wherein
the output unit further outputs the blood cell count measured by the blood cell count measurement unit.

10. The blood test apparatus according to any one of claims 1 to 9, further comprising:
an aggregation accelerator addition unit that adds a substance for promoting reversible erythrocyte aggregation to the blood sample.

11. A blood test method, comprising:
a step (a) of controlling to add a reagent to a blood sample;
a step (b) of controlling to supply the blood sample added with the reagent to a flow path;
a step (c) of measuring a degree of erythrocyte aggregation of the blood sample in the flow path; and
a step (d) of determining a blood type of the blood sample based on a change in the degree of erythrocyte aggregation of the blood sample, wherein
the reagent contains an antibody that generates antigen-antibody reaction with an antigen contained in the blood sample, or an antigen that generates antigen-antibody reaction with an antibody contained in the blood sample.

12. The blood test method according to claim 11, further comprising:
a step (e) of supplying the blood sample to the flow path without adding the reagent and measuring the degree of erythrocyte aggregation of the blood sample before the step (a), wherein
in the step (d), the blood type of the blood sample is determined based on a change in the degree of aggregation of the blood sample before and after adding the reagent to the blood sample.

13. The blood test method according to claim 12, wherein
in the steps (c) and (e), light is emitted from an irradiation unit to the flow path, transmitted light generated by the light emitted from the irradiation unit passing through the flow path is received, and the degree of erythrocyte aggregation of the blood sample is measured based on intensity of the transmitted light.

14. The blood test method according to claim 13, wherein
the reagent is a blood type antibody reagent containing an antibody that generates antigen-antibody reaction with an antigen in erythrocytes, and
in the steps (c) and (e), control is performed to allow the blood sample to flow at a predetermined flow rate at which reversible aggregation of the erythrocytes contained in the blood sample is released and then to stop the flowing of the blood sample before and after adding the reagent to the blood sample, and the degree of erythrocyte aggregation of the blood sample is measured based on a time course of the intensity of the transmitted light after the flowing of the blood sample is stopped.

15. The blood test method according to claim 14, wherein
in the steps (c) and (e), the degree of erythrocyte aggregation of the blood sample is measured based on a difference between a maximum value and a minimum value of the intensity of the transmitted light after the flowing of the blood sample is stopped.

16. The blood test method according to any one of claims 11 to 13, wherein
in the step (a), a blood type antibody reagent that generates antigen-antibody reaction with an antigen in the erythrocytes contained in the blood sample is added to the blood sample.

17. The blood test method according to any one of claims 11 to 13, wherein
in the step (a), a blood type antigen reagent that generates antigen-antibody reaction with an antibody in blood plasma contained in the blood sample is added to the blood sample.

18. The blood test method according to any one of claims 11 to 17, further comprising:
a step (f) of outputting at least one of the blood type determined in the step (d), accuracy of the blood type determination, the degree of erythrocyte aggregation of the blood sample, and an error during measurement of the degree of erythrocyte aggregation.

19. The blood test method according to claim 12, further comprising:
a step (g) of adding a substance for promoting reversible erythrocyte aggregation to the blood sample before the step (e), and after the step (a) and before the step (b).

20. A blood test program for causing a computer to execute the blood test method according to any one of claims 11 to 19.
